# EUROPEAN PATENT SPECIFICATION

(11) **EP 1 285 633 B1**
(45) Date of publication and mention of the grant of the patent: **13.12.2006**
(21) Application number: 02102099.5
(22) Date of filing: 06.08.2002
(51) Int. Cl.: A61B 19/00

(54) **Device for connecting a surgical instrument to a stable basis**
Gerät zur Verbindung eines chirurgischen Geräts mit einer stabilen Basis
Outil pour la connexion d'un instrument chirurgical avec une base stable

(30) Priority: 07.08.2001 NL 1018719
(43) Date of publication of application: 26.02.2003
(73) Proprietor: Universitair Medisch Centrum Utrecht, 3584 CX Utrecht (NL); Universiteit Utrecht, 3584 CS Utrecht (NL)
(72) Inventor: Duiveman, Eduard, 3431 BW Nieuwegein (NL)
(74) Representative: Hooiveld, Arjen Jan Winfried

(56) References cited:
- EP-A- 1 093 764
- US-A- 5 251 127
- US-A- 5 413 573
- US-A- 5 682 264
- US-A- 5 785 643

## Description

The present invention relates to a device for connecting a surgical instrument to a stable basis. In particular, the invention relates to a device for connecting and stabilizing a sterile surgical instrument to an non-sterile operating table during a surgical procedure.

During surgery it is often necessary to support and stabilize a surgical instrument, such as an endoscope or retractor, in an elevated position above the operation table. In some cases operating room personnel may manually hold the surgical instrument in the desired position and move it according to the surgeon's instructions. This is, however, unsatisfactory because, in particular during extended surgical procedures, the person holding the instrument may not be able to maintain stability because of fatigue.

It is also known to attach the surgical instrument to the operating table. Operating tables are generally provided with side rails on which surgical support equipment is clamped. The non-sterile side rails are generally separated from the sterile operation element by a sterile sheet which is used to cover the patient during surgical procedures. The connection of the clamp to the rails is, however, laborious and requires considerable forces of the person attaching the instrument.

Another disadvantage is that the surgical instrument is clamped to the rails, such that the sterile sheet remains between the side rails and the clamp. However, as in some cases the need may arise for exchanging surgical instruments during surgery and due to the fact that the sheet is squeezed between the clamp and the side rail, holes or tears may be produced in the sterile sheet as a result of wear. As a consequence, the unsterile side rail is no longer insulated from the sterile surgical instrument. The unsterility may constitute a considerable hazard to the success of the surgery and the health of the patient.

Thus, the realization of a stable and reliable linkage between elements in the unsterile area and elements, such as surgical instruments, in the sterile area presents a problem.

It is an object of the invention to provide a novel device for connecting a sterile surgical instrument to a non-sterile stable basis, obviating the aforementioned problems.

This object is achieved by providing a device according to claim 1.

With the device according to the present invention surgical instruments can easily, quickly and safely in view of sterility, be connected to a stable basis, such as an operating table.

The device of the invention thus is constituted of two interconnectable parts, the first part being applied in the non-sterile field and the second part being applied in the sterile field, wherein on connecting both parts the sterility of the second part is garantueed.

According to the invention, the tapered surfaces of the projection and the cavity comprise at least one plane side surface, in order to prevent undesired rotation of the surgical instrument.

Preferably, the tapered surfaces of the projection and of the cavity are frustum-pyramidal. During surgery, the second part thus remains stationary, but can easily be exhanged during surgery. Frustum-pyramidal according to this invention refers to a truncated pyramid with at least three side surfaces. The skilled person will understand that the tapered surfaces of the projection and of the cavity may also comprise four or more side sufaces. In case rotation of the surgical instrument during surgery is desired, the tapered surfaces of the projection and of the cavity may also be frustum-conical.

For preventing the sterile cover sheet from tearing, thus breaking the sterility, the corners of the frustum-pyramidal part of the projection, seen in cross-section, are preferably cut-off or round.

Furthermore, the tapered surface of said projection and/or cavity comprises at least one recess for receiving the pleats of the sterile cover sheet when both parts are connected.

Preferably, the projection comprises a telescopic tip which can be axially moved inward against the force of biasing means. Thus, on inserting the projection into the cavity, while including the sterile cover sheet in between, tearing of the sheet by high stress is prevented.

In a further preferred embodiment, at least one of said parts preferably comprises locking means for relative fixation of said parts, in order to improve the stability and reliabilty of the connection between the stable basis and the surgical instrument.

Preferably, the cavity comprises said locking means for enclosing the projection in the cavity, thus providing a reliable fixation of the two parts.

Advantageously, the second part comprises means for mounting one or more surgical instruments. Thus, several types of surgical instruments can be mounted to one and the same second part. The second part thus constitutes a frame for mounting of the surgical instrument. Hereto, the second part may for example comprise a rail for attaching the surgical instrument. Preferably, the rail is similar to the existing side rail which is present on conventional operating tables. Thus, existing surgical instruments, which conventionally were to be connected to the side rail, can be easily, without any adjustments and additional coupling means, connected to the rail of the second part. The second part may also comprise apertures, flat zones, projections etc, for attaching specific surgical instruments. Alternatively, the second part may form an integral part of the surgical instrument itself.

Since the second part is adapted to connect a sterile surgical instrument to a non-sterile stable basis, such as an operating table, the second part should be made of a sterilizable material. The material of the second part may for example be a metal, such as stainless steel, or a plastic. The second part may also be made of a disposable material.

Furthermore, the invention relates to a method according to claim 8. Thus, a surgical instrument can easily and safely be connected to the stable basis, without risking tearing of the sterile sheet and thus breaking the sterility.

After inserting said projection in said cavity both parts are connected by gravity, providing a reliable connection between the stable basis and the surgical instrument without difficult manipulation and using any force.

The device according to the invention preferably comprises a first non-sterile part, which is connectable to the stable basis, and a second sterile part, which is connectable to the surgical instrument. The first part comprises an at least partially tapered projection, and the second part comprises an at least partially and correspondingly tapered cavity, following the shape of the exterior of the projection of the first part, for accomodating the projection while including a sterile cover sheet between the projection and the inner surface of the cavity, without substantially damaging the sheet. The second part is disposed on the projection of the first part.

The first part in the preferred embodiment consists of a support arm which may for example be clamped to the side rail of an operating table by any conventional clamp, extending vertically from said operating table. The projection of the support arm and the cavity of the second part are so shaped that the second part slides over the projection, while including the sterile cover sheet in between. Due to the vertical orientation of the two parts in relation to the stable basis a stable and reliable connection is achieved simply by gravity.

The projection comprises a telescopic tip which can be axially moved inward against the force of biasing means, such as a spring. Thus tearing of the sheet by high stress is prevented when the projection is inserted into the cavity.

The tapered surfaces of the projection and the cavity are frustum-pyramidal, with four side surfaces, in order to prevent undesired rotation of the surgical instrument. On positioning the second part with its frustum-pyramidical inner surface on the frustum-pyramidical projection of the first part, the second part slidingly engages the surface of the projection, until the second part comes to rest on the first part.

For preventing the sterile cover sheet from tearing, thus breaking the sterility, the corners of the frustum-pyramidal part of the projection are rounded.

The tapered surface of said projection comprises number of recesses for receiving the pleats of the sterile cover sheet when both parts are connected. The second part further comprises a lock, for enclosing the projection in the cavity.

## Claims

1. Device for connecting a sterile surgical instrument to a non-sterile stable basis, comprising a first non-sterile part, which is connectable to the stable basis, and a second sterile part, which is connected or connectable to the surgical instrument, **characterized in that** one of said parts comprises an at least partially tapered projection and the other of said parts comprises an at least partially and correspondingly tapered cavity for accomodating the projection while including a sterile cover sheet between the projection and the cavity without substantially damaging the sheet, wherein the tapered surface of the projection and the tapered surface of the cavity comprises at least one plane side surface, further **characterized in that** the tapered surface of said projection and/or cavity comprises at least one recess.

2. Device as claimed in claim 1, **characterized in that** the tapered surfaces of the projection and the cavity are frustum-pyramidal

3. Device as claimed in claim 2, **characterized in that** the corners of the frustum-pyramidal part of the projection, seen in cross-section, are cut-off or round.

4. Device as claimed in claim 1, 2 or 3, **characterized in that** the projection comprises a telescopic tip which can be axially moved inward against the force of biasing means.

5. Device as claimed in any of the preceding claims 1-4, **characterized in that** at least one of said parts comprises locking means for relative fixation of said parts.

6. Device as claimed in 5, **characterized in that** the part comprising the cavity comprises said locking means for enclosing the projection in the cavity.

7. Device as claimed in any of the preceding claims 1-5, **characterized in that** the second part comprises means for mounting one or more surgical instruments.

8. Method for connecting a sterile surgical instrument to a non-sterile stable basis, such as an operating table, wherein a first non-sterile part is connected to the stable basis, and wherein a second sterile part is connected to the surgical instrument, **characterized in that** one of said parts comprises an at least partially tapered projection and the other of said parts comprises an at least partially and correspondingly tapered cavity and wherein the tapered surface of said projection and/or cavity comprises at least one recess, wherein the projection is inserted in the cavity while including a sterile cover sheet between the projection and the cavity, further **characterized in that** after inserting said projection in said cavity both parts are connected by gravity.

## Patentansprüche

1. Gerät zur Verbindung eines sterilen chirurgischen Instruments mit einer nichtsterilen stabilen Basis, umfassend einen ersten nichtsterilen Teil, der mit der stabilen Basis verbindbar ist, und einen zweiten sterilen Teil, der mit dem chirurgischen Instrument verbunden oder verbindbar ist, **dadurch gekennzeichnet, daß** einer der Teile einen mindestens teilweise verjüngten Vorsprung umfaßt und der andere der Teile einen wenigstens teilweise und entsprechend verjüngten Hohlraum zur Aufnahme des Vorsprungs während Zwischenschaltung einer sterilen Abdeckung zwischen dem Vorsprung und dem Hohlraum ohne wesentliche Beschädigung der Abdeckung umfaßt, worin die verjüngte Fläche des Vorsprungs und die verjüngte Fläche des Hohlraums wenigstens eine ebene Seitenfläche umfaßt, **dadurch gekennzeichnet, daß** die verjüngte Fläche des Vorsprungs und/oder des Hohlraums wenigstens eine Aussparung umfaßt/umfassen.

2. Gerät nach Anspruch 1, **dadurch gekennzeichnet, daß** die verjüngten Flächen des Vorsprungs und des Hohlraums pyramidenstumpfförmig sind.

3. Gerät nach Anspruch 2, **dadurch gekennzeichnet, daß** die Ecken des pyramidenstumpfförmigen Teils des Vorsprungs, im Querschnitt, abgeschnitten oder abgerundet sind.

4. Gerät nach Anspruch 1, 2 oder 3, **dadurch gekennzeichnet, daß** der Vorsprung eine Teleskopspitze umfaßt, die nach innen gegen die Kraft eines Vorspannmittels axial bewegt werden kann.

5. Gerät nach einem der vorangehenden Ansprüche 1 - 4, **dadurch gekennzeichnet, daß** wenigstens einer der Teile ein Verriegelungsmittel zur relativen Fixierung der Teile umfaßt.

6. Gerät nach Anspruch 5, **dadurch gekennzeichnet, daß** der Teil mit dem Hohlraum das Verriegelungsmittel zum Einschließen des Vorsprungs im Hohlraum umfaßt.

7. Gerät nach einem der vorangehenden Ansprüche 1-5, **dadurch gekennzeichnet, daß** der zweite Teil ein Mittel zum Befestigen von einem oder mehreren chirurgischen Instrument(en) umfaßt.

8. Verfahren zur Verbindung eines sterilen chirurgischen Instruments mit einer nichtsterilen stabilen Basis, wie zum Beispiel einem Operationstisch, worin ein erster nichtsteriler Teil mit der stabilen Basis verbunden wird, und worin ein zweiter steriler Teil mit dem chirurgischen Instrument verbunden wird, **dadurch gekennzeichnet, daß** einer der Teile einen wenigstens teilweise verjüngten Vorsprung umfaßt und der andere der Teile einen wenigstens teilweise und entsprechend verjüngten Hohlraum umfaßt und worin die verjüngte Fläche des Vorsprungs und/oder Hohlraums wenigstens eine Aussparung umfaßt, worin der Vorsprung in den Hohlraum während Zwischenschaltung einer sterilen Abdeckung zwischen dem Vorsprung und dem Hohlraum eingesetzt wird, **dadurch gekennzeichnet, daß** nach Einsetzen des Vorsprungs in den Hohlraum beide Teile durch Schwerkraft verbunden werden.

## Revendications

1. Dispositif de raccordement d'un instrument chirurgical stérile à une base stable non stérile, comprenant une première partie non stérile, qui est destinée à être raccordée à la base stable, et une seconde partie stérile, qui est raccordée ou est destinée à être raccordée à l'instrument chirurgical, **caractérisé en ce que** l'une des parties comporte une saillie au moins partiellement effilée et l'autre des parties comprend une cavité au moins partiellement effilée de manière correspondante pour le logement de la saillie tout en comprenant une feuille stérile de couverture placée entre la saillie et la cavité sans pratiquement détérioration de la feuille, dans lequel la surface effilée de la saillie et la surface effilée de la cavité comportent au moins une surface latérale plane, et **caractérisé en outre en ce que** la surface effilée de la saillie et/ou de la cavité comporte au moins un évidement.

2. Dispositif selon la revendication 1, **caractérisé en ce que** les surfaces effilées de la saillie et de la cavité sont en tronc de pyramide.

3. Dispositif selon la revendication 2, **caractérisé en ce que** les coins de la partie en tronc de pyramide de la saillie, vue en coupe, sont cassés ou arrondis.

4. Dispositif selon la revendication 1, 2 ou 3, **caractérisé en ce que** la saillie a un bout télescopique qui peut être déplacé axialement vers l'intérieur malgré la force d'un dispositif de rappel.

5. Dispositif selon l'une quelconque des revendications 1 à 4, **caractérisé en ce que** l'une au moins des parties comprend un dispositif de blocage destiné à une fixation relative des parties.

6. Dispositif selon la revendication 5, **caractérisé en ce que** la partie comprenant la cavité comporte le dispositif de blocage destiné à entourer la saillie placée dans la cavité.

7. Dispositif selon l'une quelconque des revendications précédentes 1 à 5, **caractérisé en ce que** la seconde partie comporte un dispositif de montage d'un ou plusieurs instruments chirurgicaux.

8. Procédé de raccordement d'un instrument chirurgical stérile à une base stable non stérile, telle qu'une table d'opération, dans lequel une première partie non stérile est raccordée à la base stable, et dans lequel une seconde partie stérile est raccordée à l'instrument chirurgical, **caractérisé en ce que** l'une des parties comprend une saillie au moins partiellement effilée et l'autre des parties comprend une cavité au moins partiellement effilée de manière correspondante, la surface effilée de la saillie et/ou de la cavité comprenant au moins un évidement, tel que la saillie est insérée dans la cavité en comprenant une feuille stérile de couverture entre la saillie et la cavité, et **caractérisé en outre en ce que**, après insertion de la saillie dans la cavité, les deux parties sont raccordées sous l'action de la pesanteur.
